Europäisches Patentamt

European Patent Office

Office européen des brevets

(19)

(11) Veröffentlichungsnummer: **0 242 794**
A2

(12) # EUROPÄISCHE PATENTANMELDUNG

(21) Anmeldenummer: 87105610.7

(22) Anmeldetag: 15.04.87

(51) Int. Cl.³: **A 61 K 7/04**
A 61 K 7/06, A 61 K 7/48
A 61 K 7/50

(30) Priorität: 22.04.86 DE 3613525
08.08.86 DE 3626913

(43) Veröffentlichungstag der Anmeldung:
28.10.87 Patentblatt 87/44

(84) Benannte Vertragsstaaten:
AT BE CH DE ES FR GB GR IT LI LU NL SE

(71) Anmelder: Allnatur Bio-Produkte GmbH
Bockumerstrasse 171
D-4000 Düsseldorf 31(DE)

(72) Erfinder: Blöst, Elly
Meineckestrasse 61
D-4000 Düsseldorf 30(DE)

(72) Erfinder: Welkert, Ute
Goethestrasse 7a
D-4030 Ratingen 1(DE)

(74) Vertreter: Werner, Hans-Karsten, Dr. et al,
Deichmannhaus am Hauptbahnhof
D-5000 Köln 1(DE)

(54) Lagerfähige Zubereitungen und Verfahren zur Herstellung derselben.

(57) Lagerfähige Zubereitungen, enthaltend die Inhaltsstoffe frisch gewonnener Weizenschlempe, werden hergestellt, indem gegebenenfalls die Spelzen entfernt werden, dann kolloidal vermahlen und schonend getrocknet wird, wobei gegebenenfalls Fließmittel, Netzmittel, Aromastoffe sowie weitere pulverförmige oder ölige Pflanzenextrakte zugegeben werden. Die schonende Trocknung erfolgt vorzugsweise durch Sprühtrocknung.

EP 0 242 794 A2

-1-

## Lagerfähige Zubereitungen und Verfahren zur Herstellung derselben

Gegenstand der vorliegenden Erfindung sind lagerfähige Zubereitungen, enthaltend die Inhaltsstoffe frisch gewonnener Weizenschlempe, und Verfahren zur Herstellung derselben.

Im Kurhaus von Rheindahlen sind seit Jahren erfolgreiche Behandlungen mit Weizenschlammbädern durchgeführt worden, die sich insbesondere bei der Behandlung von Rheuma- und Hauterkrankungen bewährt haben. Erstaunliche Erfolge wurden insbesondere auch bei Psoriasis-Patienten erzielt sowie bei Patienten mit Gelenkbeschwerden. Verwendet wurden für diese Weizenschlammbäder ausschließlich frisch gewonnene heiße Schlempen aus der Rheindahlener Kornbrennerei, der Hans Rother Kornbrennerei und Likörfabrik in Werne. Hierbei handelt es sich um Weizenschlempen, die schonend destilliert wurden, nämlich durch Abtreiben des Alkohols durch Einblasen von Dampf in die Schlempe. Insbesondere handelt es sich um Weizenschlempen, die nach dem sogenannten Bona-Destillata-Brennverfahren gemäß DE-PS 20 50 212 anfallen.

Ein wesentlicher Nachteil dieser Weizenschlammbäder und ihrer breiteren Anwendung besteht darin, daß hierfür stets heiße und relativ frische Weizenschlempe verwendet werden muß, da beim Abkühlen die Gefahr von bakteriellen Infektionen besteht und bei längerer Lagerung bei hohen Temperaturen die wertvollen Bestandteile abgebaut oder zerstört werden, so daß die Wirksamkeit nachläßt.

- 2 -

Die Erfindung hat sich die Aufgabe gestellt, lagerfähige Zubereitungen zu entwickeln, enthaltend die Inhaltsstoffe frisch gewonnener Weizenschlempe, so daß sie praktisch an jedem beliebigen Ort und zu jeder beliebigen Zeit eingesetzt werden können. Dabei war darauf zu achten, daß die wertvollen Bestandteile ebenfalls erhalten und stabilisiert werden, so daß diese Zubereitungen zumindest vergleichbar bleiben mit Weizenschlammbädern aus frischer heißer Weizenschlempe. Eingehende Untersuchungen haben schließlich dazu geführt, daß diese Aufgabe gelöst werden kann, indem man die frisch gewonnene Weizenschlempe gegebenenfalls von den Spelzen befreit, kolloidal vermahlt und dann schonend trocknet. Das so erhaltene Produkt wird gegebenenfalls mit Fließmitteln, Netzmitteln, Aromastoffen sowie weiteren pulverförmigen oder öligen Pflanzenextrakten vermischt. Es kann in dieser Form abgepackt, gelagert, transportiert und jederzeit für Bäder eingesetzt werden, indem man diese Zubereitung wieder mit heißem Wasser vermischt. Das so erhaltene Produkt kann aber auch in die Form von Hautcreme, Gel, Shampoo, Gesichtsmasken aus Kollagen oder Brausetabletten gebracht werden.

Gegenstand der vorliegenden Erfindung sind somit die lagerfähigen Zubereitungen enthaltend die Inhaltsstoffe frisch gewonnener Weizenschlempe, dadurch gekennzeichnet, daß sie gegebenenfalls von den Spelzen befreite, kolloidal vermahlene, schonend getrocknete Weizenschlempe sowie gegebenenfalls Fließmittel, Netzmittel, Aromastoffe sowie weitere pulverförmige oder ölige Pflanzenextrakte enthalten. Vorzugsweise wird als Weizenschlempe eine schonend destillierte Weizenschlempe verwendet, die erhalten wird durch Abtreiben des Alkohols durch Einleiten mit Dampf. Besonders bevorzugt ist Weizenschlempe, die nach dem sogenannten Bona-Destillata-Verfahren bzw. gemäß Patent 20 50 212 anfällt.

Insbesondere um die Rieselfähigkeit des getrockneten Pulvers für Bäder über längere Zeit zu gewährleisten, wird ihm vorzugsweise ein Fließmittel beigefügt, wobei sich Gemische aus Tricalciumphosphat und Kieselsäure besonders bewährt haben. Diese Fließmittel werden in Mengen von 1 bis 5%, vorzugsweise 2%, dem Pulver beigemischt. Dem Pulver können weiterhin gewünschtenfalls pulverförmige oder ölige Pflanzenextrakte beigemischt werden. Insbesondere eignet sich hierfür Kamillenextraktpulver. Weitere geeignete Zusätze sind Rosmarin, Melisse, Zitronenkampfer, Menthol, Weizenkeimöl, Sojaöl und Lecithin. Der Zusatz von festen Tensiden wie Natriumlaurylsulfat sollte auf relativ kleine Mengen beschränkt werden, da anderenfalls die Wirksamkeit der Inhaltsstoffe der Weizenschlempe vermindert wird.

Insbesondere um die wertvollen Inhaltsstoffe der Weizenschlempe zu erhalten, soll nicht nur schonend destilliert werden, sondern muß insbesondere danach schonend getrocknet werden. Als besonders geeignet hat sich die Sprühtrocknung erwiesen, bei der sich aus einer 5%-igen Schlempe in einem Arbeittschritt und ohne lange thermische Belastung ein trockenes Pulver erzielen läßt, welches noch 2 bis 4% Restfeuchte enthält. Da dieses so entstandene Produkt nach einiger Zeit dazu neigt, wieder zu verklumpen und seine Rieselfähigkeit zu verlieren, wird daher vorzugsweise dem Gemisch ein Fließmittel beigemischt. Zur Verstärkung der Wirksamkeit ist es möglich, diesen Produkten auch noch weitere pulverförmige oder ölige Pflanzenextrakte beizumischen.

Für die Zubereitung eines Bades aus dem erfindungsgemäß hergestellten pulverförmigen Produkt genügt es beispielsweise, 75 g in die Badewanne zu geben und ca. 35 bis 40°C warmes Wasser zulaufen zu lassen.

Um die Wirkung zu verstärken, können aber ohne weiteres auch pro Bad 2 oder 3 Beutel à 75 g eingesetzt werden. Das Bad sollte dann ca. 20 bis 25 Minuten einwirken. Genau wie nach den Weizenschlammbädern mit frisch zubereiteter Weizenschlempe sollte nach dem Bad ca. eine Stunde geruht werden.

Vergleichende Untersuchungen der erfindungsgemäßen trockenen, lagerfähigen Zubereitung für Bäder haben ergeben, daß der Gehalt an Vitaminen, Aminosäuren und Spurenelementen nahezu derselbe ist wie bei Weizenschlammbädern mit frisch gewonnener, unvermahlener Weizenschlempe. Versuche mit unvermahlener Schlempe sowie Weizenschlempe, die auf Walzentrocknern getrocknet wurde, haben hingegen gezeigt, daß hieraus zubereitete Bäder wesentlich weniger wirksam sind und wesentlich geringere Mengen an wertvollen Wirkstoffen enthalten.

Insbesondere für die Herstellung von Hautcreme, Gel, Shampoo, Gesichtsmasken aus Kollagen oder Brausetabletten empfiehlt es sich, vor dem kolloidalen Vermahlen die Spelzen abzutrennen. Dies erfolgt am einfachsten durch Dekantieren. Es hat sich nämlich überraschenderweise gezeigt, daß die Spelzen sich wesentlich schlechter kolloidal vermahlen lassen, jedoch andererseits nahezu keine der wertvollen Inhaltsstoffe der frisch gewonnenen Weizenschlempe enthalten.

Zur Herstellung einer Hautcreme kann das erfindungsgemäß hergestellte getrocknete Produkt in eine übliche Grundlage für Hautcremes eingearbeitet werden. Besonders bewährt haben sich Öl-in-Wasser- und Wasser-in-Öl-Emulsionen auf Basis von Lanolin. Der Mengenanteil an erfindungsgemäß hergestellter, lagerfähiger Zubereitung beträgt 10 bis 15, vorzugsweise 10 Gew.-%.

Zur Stabilisierung haben sich insbesondere hautverträgliche Zuckertenside bewährt.

Zur Herstellung eines Gels können übliche pharmakologisch verträgliche Gelgrundlagen von Hydrokolloiden verwendet werden. Da diese Gele meist als Packung angewendet werden, kann auch von Weizenschlempe ausgegangen werden, die noch die Spelzen enthält. Vorzugsweise wird aber auch hierfür ein Material eingesetzt, das vorher von den Spelzen befreit wurde. Der Gehalt des Gels kann im Bereich zwischen 5 und 25 Gew.-% gewählt werden, da die Packungen anschließend wieder abgewaschen werden.

Zur Herstellung eines Haarshampoos können ebenfalls bis zu 10 Gew.-% von Spelzen befreite, kolloidal vermahlene und schonend getrocknete Weizenschlempe verwendet werden. Auch hier kommen als Netzmittel Zuckertenside, aber auch andere hautverträgliche Detergenzien in Frage.

Eine weitere interessante Anwendungsform sind Gesichtsmasken aus Kollagen, in welche bereits bei der Herstellung der Masken die erfindungsgemäßen Zubereitungen eingearbeitet werden. Erfindungsgemäß verwendbare Gesichtsmasken aus Kollagen werden bisher ohne derartige Zusätze angeboten von der Dr. Suwelack GmbH in Billerbeck. Geeignete schwammartige Kollagen-Zubereitungen sind beispielsweise beschrieben in der DE-OS 32 03 957. Auch in derartige Gesichtsmasken können 3 bis 15 Gew.-% der erfindungsgemäßen Zubereitungen eingearbeitet werden. Die Kollagenmasken werden kurz vor der Anwendung angefeuchtet und dann auf die Gesichtshaut aufgelegt. Sie gestatten ein gleichmäßiges Eindringen der wirksamen Bestandteile der Weizenschlempe in die darunterliegende Haut.

Schließlich ist es möglich, die lagerfähigen Zubereitungen in die Form von Brausetabletten zu bringen, so daß es möglich ist, die erfindungsgemäßen Produkte auf engstem Raum zu verpacken und zu lagern. Beim Auflösen der Brausetabletten werden die wirksamen Bestandteile wieder freigesetzt und können dann in einem sprudelnden und schäumenden Bad zur Wirkung kommen. Derartige Brausetabletten sind insbesondere für Bäder für Kleinkinder sowie für den Export geeignet. Die sprudelnde Wirkung der Brausetabletten wird in üblicher Weise erzielt durch ein festes Alkalibicarbonat und eine feste Säure wie Zitronensäure, die, räumlich voneinander getrennt, miteinander zu Tabletten verpreßt werden.

Untersuchungen mit den erfindungsgemäßen Zubereitungen haben gezeigt, daß nicht nur bei Anwendung von Bädern, sondern auch von Hautcreme, Gel, Shampoo, Gesichtsmasken aus Kollagen und Brausetabletten erstaunliche Heil- und Linderungserfolge eintreten. Die erfindungsgemäßen Zubereitungen können somit nicht nur zur allgemeinen Hautpflege und zur Vorbeugung, sondern auch bei der Heilung von rheumatischen Erkrankungen, Psoriasis, Arthrose, Akne, Venenentzündung und Krampfadern, Blutergüssen und Verstauchungen eingesetzt werden.

Insbesondere für die Zubereitung von Hautcreme und Gel hat es sich bewährt, Sorbinsäureozonid als weitere Komponente zuzumischen. Sorbinsäureozonid führt zur Freisetzung von aktivem Sauerstoff und ist in der Lage, die Wirksamkeit der übrigen Bestandteile zu verstärken. Es wurde weiterhin eine positive Wirkung auf die Lagerfähigkeit und Langzeitstabilität von Cremes und Gelen festgestellt.

In den nachfolgenden Beispielen sind Herstellung und Zusammensetzung der erfindungsgemäßen Zubereitungen näher erläutert:

B e i s p i e l  1

Frisch gewonnene, heiße Weizenschlempe aus der Rheindahlener Kornbrennerei, die bei dem sogenannten Bona-Destillata-Verfahren gemäß DE-PS 20 50 212 anfällt und einen Feststoffgehalt von 4 bis 5% hat, wurde kolloidal vermahlen und anschließend sprühgetrocknet. Die Restfeuchte betrug 2 bis 4%. Nach dem Trocknungsprozeß wurden 2% Fließmittel und 0,1% Kamillenextraktpulver beigemischt. Das Fließmittel war eine 50%-ige Mischung aus Tricalciumphosphat und Kieselsäure. Die so erhaltene Zubereitung wurde in Plastiktüten mit je 75 g Inhalt abgefüllt und eingesiegelt. Die trockene Zubereitung war mehrere Monate lagerfähig, ohne sich qualitativ zu verändern.

Je 1, 2 oder 3 Plastikbeutel wurden zur Anwendung in eine Badewanne gegeben und mit 35 bis 40°C heißem Wasser gefüllt. Die Wirksamkeit dieser Bäder war völlig vergleichbar mit Weizenschlammbädern unter Verwendung frischer, heißer Weizenschlempe.

Die analytische Untersuchung der erfindungsgemäßen trockenen, lagerfähigen Zubereitung ergab folgende Werte: 100 g Zubereitung enthielten 30 I.E. Vitamin A und 0,16 mg Vitamin E (Gesamt-Tocopherol). Weiterhin wurden gefunden 11,5 mg Vitamin $B_1$, 44 mg Vitamin $B_2$, 28 mg Vitamin $B_6$, 0,84 mg Nikotinamid, 0,115 mg Pantothensäure, 2,45 mg Biotin und 5,7 mg Folsäure.

An freien Aminosäuren wurden gefunden (Konzentration in Mikromol/ml):

| | |
|---|---|
| Taurin | 0,537 |
| Asparaginsäure | 0,397 |
| Threonin | 0,223 |
| Serin | 0,549 |
| Asparagin | 0,097 |
| Glutaminsäure | 0,769 |
| Glutamin | 0,039 |
| Glycin | 0,337 |
| Alanin | 0,960 |
| Valin | 0,441 |
| Cystin | 0,116 |
| Methionin | $<$ 0,005 |
| Isoleucin | 0,283 |
| Leucin | 0,789 |
| Tyrosin | 0,069 |
| Phenylalanin | 0,272 |
| Ornithin | 0,469 |
| Lysin | 0,415 |
| Histidin | 0,165 |
| 3-Methylhistidin | $\sim$ 0,07 |
| Tryptophan | $\sim$ 0,07 |
| Arginin | $<$ 0,005 |
| Cysteinsäure | $\sim$ 1,000 |

Folgende Spurenelemente und Elektrolyte wurden bestimmt:

| | | | | |
|---|---|---|---|---|
| Co | ( 1,50 $\pm$ 0,25) | $\cdot 10^{-9}$ | g pro g Probe | |
| Se | ( 5,80 $\pm$ 1,84) | " | " | |
| Sb | ( 6,24 $\pm$ 1,40) | " | " | |
| Cd | ( 6,40 $\pm$ 0,90) | " | " | |
| As | ( 12,40 $\pm$ 1,80) | " | " | |
| Pb | ( 26,00 $\pm$ 2,40) | " | " | |
| Rb | ( 0,26 $\pm$ 0,05) | $\cdot 10^{-6}$ | " | |
| Zn | ( 3,83 $\pm$ 0,35) | " | " | |
| Fe | ( 4,58 $\pm$ 0,42) | " | " | |
| Mn | ( 43,00 $\pm$ 4,50) | " | " | |
| Na | ( 52,00 $\pm$ 14,80) | " | " | |
| K | (933,00 $\pm$ 185,00) | " | " | |

- 9 -

Die Elemente As, Pb und Cd wurden mit Hilfe der Atomabsorptionsspektrometrie und die anderen 9 Elemente neutronenaktivierungsanalytisch ermittelt.

Untersuchungen an Patienten mit Rheuma und Psoriasis sowie beiden Krankheitsbildern wurden vorgenommen. 96% der Rheumakranken waren nach 6 bis 12 Bädern völlig beschwerdefrei, verspürten eine deutliche Besserung oder fanden eine spürbare Linderung. Nur 3,9% der Patienten berichteten über keine Besserung. Bei Psoriasis vulgaris waren 6% nach 10 Bädern völlig beschwerdefrei, 37% verspürten eine deutliche Besserung und die restlichen Patienten berichteten zumindest über eine spürbare Besserung. Bei Patienten, die unter Rheuma und Psoriasis vulgaris litten, war die Behandlung in 89% der Fälle erfolgreich, und zwar sowohl bezüglich Psoriasis als auch der rheumatischen Beschwerden.

B e i s p i e l 2

Frisch gewonnene, heiße Weizenschlempe aus der Rheindahlener Kornbrennerei, die bei dem sogenannten Bona-Destillata-Verfahren gemäß DE-PS 20 50 212 anfällt und einen Feststoffgehalt von 4 bis 5% hat, wurde dekantiert, wobei die Spelzen nahezu vollständig abgetrennt wurden. Der verbleibende Rest wurde kolloidal vermahlen und anschließend sprühgetrocknet. Die Restfeuchte betrug 3 bis 4%. Das so erhaltene Produkt wurde in folgender Weise weiterverarbeitet:

Hautcreme: Eine handelsübliche Hautcreme auf Basis einer Emulsion Lanolin in Wasser wurde mit 10% des sprühgetrockneten Produktes und 1 Gew.-% eines Zuckertensides intensiv verrührt. Man erhielt eine gut einreibbare Hautcreme.

Haarshampoo: Ein handelsüblicher Haarshampoo wurde mit 8 Gew.-% des sprühgetrockneten Produktes und 1% Zuckertensid verrieben. Man erhielt einen gut schäumenden Haarshampoon.

Gel: Eine handelsübliche Gelgrundlage (Hydrokolloid) wurde mit 15 Gew.-% des sprühgetrockneten Produktes vermischt. Man erhielt eine gut auftragbare Paste, die als Packung auf Krampfadern etc. aufgebracht werden konnte.

Gesichtsmaske aus Kollagen: In die Grundrezeptur für Gesichtsmasken aus Kollagen der Firma Dr. Suwelack GmbH in Billerbeck wurden 10% des sprühgetrockneten Produktes eingemischt und in üblicher Weise zu Gesichtsmasken verarbeitet. Die Gesichtsmasken hatten mechanisch die gleichen Eigenschaften wie die bisherigen Handelsprodukte, enthielten jedoch die Wirkstoffe frischer Weizenschlempe. Derartige Gesichtsmasken führten zu einer deutlichen Straffung und Regeneration der Haut.

Brausetabletten: Eine handelsübliche Grundmischung für Brausetabletten, enthaltend Natriumbicarbonat und Zitronensäure, wurde mit 10 Gew.-% gefriergetrocknetem Produkt und 5% Zuckertensiden vermischt. Nach dem Einwerfen in warmes Wasser entstand ein sprudelndes und schäumendes Bad mit den Inhaltsstoffen frischer Weizenschlempe.

— *11* —

P a t e n t a n s p r ü c h e

1. Lagerfähige Zubereitungen enthaltend die Inhaltsstoffe frisch gewonnener Weizenschlempe, dadurch gekennzeichnet, daß sie gegebenenfalls von den Spelzen befreite, kolloidal vermahlene, schonend getrocknete Weizenschlempe sowie gegebenenfalls Fließmittel, Netzmittel, Aromastoffe sowie weitere pulverförmige oder ölige Pflanzenextrakte enthalten.

2. Zubereitungen gemäß Anspruch 1, dadurch gekennzeichnet, daß als Weizenschlempe eine schonend destillierte Weizenschlempe verwendet wird, die erhalten wird durch Abtreiben des Alkohols durch Einleitung von Dampf.

3. Zubereitungen gemäß Ansprüchen 1 oder 2, dadurch gekennzeichnet, daß die schonende Trocknung durch Sprühtrocknung erfolgt.

4. Zubereitungen gemäß Ansprüchen 1 oder 2, dadurch gekennzeichnet, daß die schonende Trocknung durch Gefriertrocknung erfolgt.

5. Zubereitungen gemäß einem der Ansprüche 1 bis 4, dadurch gekennzeichnet, daß sie in Form von Hautcreme, Gel, Shampoo, Gesichtsmasken aus Kollagen oder Brausetabletten vorliegen.

6. Trockene, lagerfähige Zubereitung für Bäder, bestehend aus kolloidal vermahlener und schonend getrockneter Weizenschlempe, gegebenenfalls einem Fließmittel sowie weiteren pulverförmigen oder öligen Pflanzenextrakten.

7. Zubereitungen gemäß Anspruch 6, dadurch gekennzeichnet, daß als Weizenschlempe eine schonend destillierte Weizenschlempe verwendet wird, die erhalten wird durch Abtreiben des Alkohols durch Einleitung von Dampf.

8. Verfahren zur Herstellung von lagerfähigen Zubereitungen enthaltend die Inhaltsstoffe frisch gewonnener Weizenschlempe, dadurch gekennzeichnet, daß Weizenschlempe gegebenenfalls nach Abtrennung der Spelzen kolloidal vermahlen, schonend getrocknet und das so erhaltene Produkt gegebenenfalls mit einem Fließmittel, Netzmittel, Aromastoff sowie weiteren pulverförmigen oder öligen Pflanzenextrakten vermischt wird.

9. Verfahren gemäß Anspruch 8, dadurch gekennzeichnet, daß als Weizenschlempe eine schonend destillierte Weizenschlempe verwendet wird, die erhalten wird durch Abtreiben des Alkohols durch Dampf.

10. Verfahren gemäß einem der Ansprüche 8 oder 9, dadurch gekennzeichnet, daß die schonende Trocknung durch Sprühtrocknung erfolgt.

11. Verfahren gemäß Ansprüchen 8 oder 9, dadurch gekennzeichnet, daß die schonende Trocknung durch Gefriertrocknung erfolgt.

12. Verfahren gemäß einem der Ansprüche 9 bis 11, dadurch gekennzeichnet, daß das so erhaltene Produkt weiterverarbeitet wird zu Hautcreme, Gel, Shampoo, Gesichtsmasken aus Kollagen oder Brausetabletten.

13. Verfahren zur Herstellung einer trockenen, lagerfähigen Zubereitung für Bäder, dadurch gekennzeichnet, daß Weizenschlempe kolloidal vermahlen und dann schonend getrocknet wird und das so erhaltene Produkt gegebenenfalls mit einem Fließmittel sowie weiteren pulverförmigen oder öligen Pflanzenextrakten vermischt wird.

14. Verfahren gemäß Anspruch 13, dadurch gekennzeichnet, daß als Weizenschlempe eine schonend destillierte Weizenschlempe verwendet wird, die erhalten wird durch Abtreiben des Alkohols durch Dampf.